# EUROPÄISCHE PATENTSCHRIFT

(11) **EP 2 272 819 B1**
(45) Veröffentlichungstag und Bekanntmachung des Hinweises auf die Patenterteilung: **26.11.2014**
(21) Anmeldenummer: 10006116.7
(22) Anmeldetag: 12.06.2010
(51) Int. Cl.: C07C 68/06, C07C 69/96, C07C 29/128, B01D 3/00

(54) **Verfahren zur Herstellung von Dialkylcarbonaten aus Alkylencarbonaten und Alkoholen**
Method for producing dialkylcarbonates from alkyl carbonates and alcohols
Procédé de fabrication de carbonates de dialkyle à partir de carbonates d'alkyle et d'alcools

(30) Priorität: 26.06.2009 DE 102009030680
(43) Veröffentlichungstag der Anmeldung: 12.01.2011
(73) Patentinhaber: Bayer Intellectual Property GmbH, 40789 Monheim (DE)
(72) Erfinder: Risse, Friedhelm, Dr., 50739 Köln (DE); Ooms, Pieter, Dr., 47800 Krefeld (DE); Düx, Andre, 50321 Brühl (DE); Grünewald, Marcus, Dr., 44309 Dortmund (DE); Pancur, Thomas, Dr., 24161 Altenholz (DE); Susanto, Arthur, Dr., 50670 Köln (DE); Ronge, Georg, Dr., 40549 Düsseldorf (DE); Vanden Eynde, Johan, 9052 Zwijnaarde (BE); Wuytack, Wim, 9240 Zele (BE)
(74) Vertreter: BIP Patents

(56) Entgegenhaltungen:
- EP-A1- 0 569 812
- EP-A1- 1 967 242
- WO-A1-2007/096342
- WO-A1-2007/096343

## Beschreibung

Die vorliegende Erfindung betrifft ein kontinuierliches Verfahren zur Herstellung von niederen Dialkylcarbonaten als Hauptprodukt und Alkylenglykol als Nebenprodukt durch katalysierte Umesterung eines zyklischen Alkylencarbonats (z.B. Ethylen- oder Propylencarbonat) mit niederen Alkoholen, wobei die Umsetzung des Alkylencarbonats mit einem Dialkylcarbonat enthaltenden Alkohol im Gegenstrom vorgenommen wird, **dadurch gekennzeichnet, dass** eine Einführung eines Stroms enthaltend mindestens 99,5 Gew.-% Alkohol unterhalb der Einführungsstelle für den Dialkylcarbonat enthaltenden Alkohol in einem bestimmten Abstandsverhältnis zwischen den bereits genannten Einfiihrungsstellen stattfindet.

Die Herstellung von Dialkylcarbonaten aus zyklischem Alkylencarbonat und Alkohol, bei der gleichzeitig Alkylenglykol als Nebenprodukt entsteht, ist bekannt und vielfach beschrieben worden. In US 6,930,195 B2 wurde diese katalysierte Umesterungsreaktion als zweistufige Gleichgewichtsreaktion beschrieben. In der ersten Reaktionsstufe reagiert das zyklische Alkylencarbonat mit Alkohol zu Hydroxy-alkylcarbonat als Zwischenprodukt. Das Zwischenprodukt wird dann in der zweiten Reaktionsstufe mit Hilfe von Alkohol umgesetzt zu den Produkten: Dialkylcarbonat und Alkylenglykol. Für die Entwicklung eines wirtschaftlich attraktiven Herstellungsverfahrens für Dialkylcarbonate spielt die Qualität des Nebenproduktes (Alkylenglykol) neben der Qualität des Dialkylcarbonats eine sehr wichtige und tragende Rolle. Es besteht somit ein dringender Bedarf an einem Herstellungsverfahren, das das Alkylenglykol mit einem möglichst niedrigen Verunreinigungsgehalt herstellen kann.

Für die technische Realisierung des Dialkylcarbonat-Herstellungsverfahrens hat sich die Verwendung einer Reaktivdestillationskolonne, die unter anderem bereits in EP 569 812 A und EP 1 086 940 A beschrieben wurde, als besonders günstig erwiesen. In EP 569 812 A wird das zyklische Alkylencarbonat in den oberen Teil der Kolonne und der Dialkylcarbonat enthaltender Alkohol in den mittleren oder unteren Teil der Kolonne kontinuierlich eingeführt. Zusätzlich wird unterhalb der Einführung des Dialkylcarbonat enthaltenden Alkohols reiner Alkohol eingeführt. Das Leichtsiedergemisch, welches das hergestellte Dialkylcarbonat beinhaltet, wird am Kolonnenkopf und das Schwersiedergemisch, welches das hergestellte Alkylenglykol beinhaltet, wird am Kolonnensumpf kontinuierlich abgezogen.

In EP 1 086 940 wurde die Herstellung des Dialkylcarbonats auch mit Hilfe einer Reaktivdestillationskolonne demonstriert. Dabei ist die Anordnung der Edukteinführung- und Produktentnahmestelle entlang der Reaktivdestillationskolonne ähnlich der in EP 569 812, außer dass man hier auf die zusätzliche Einführung von reinem Alkohol im unteren Kolonnenbereich verzichtet hat.

EP 1 967 242 A1 beschreibt ein Verfahren zur Herstellung von einem Dialkylcarbonat und einem Diol aus einem zyklischen Carbonat und einem aliphatischen Alkohol indem diese Edukte kontinuierlich einer mehrstufigen Destillationskolonne zugeführt werden. Jedoch werden die Methanol-Zufuhren mit verschiedenen Zusammensetzungen gemeinsam in der Stufe 31 eingespeist.

Es wurde ermittelt, dass die Schwierigkeiten bezüglich der Einhaltung der hohen Qualitätsanforderung an das Nebenprodukt Alkylenglykol besonders einfach und günstig zu lösen sind, wenn der Gehalt an nicht umgesetztem Alkylencarbonat nicht erst in den nachfolgenden Aufarbeitungsschritten sondern bereits nach der Umesterungsreaktion sehr klein sind.

Es stellt sich heraus, dass die Verunreinigungen nur unter erhöhtem Aufwand (z.B. energetisch) durch die Verwendung der in EP 1 086 940 beschriebenen Anordnung der Edukteinführungsstellen reduziert werden können. Die zusätzliche Verwendung einer zweiten Alkohol-Einfiihrungsstelle unterhalb der Einführungsstelle für den Dialkylcarbonat enthaltenden Alkohol ermöglicht alleine nicht das Einhalten der gewünschten Reinheit der Produkte. Außerdem erfordert dieses offenbarte Verfahren die Verwendung von reinem Alkohol in der zweiten Alkohol-Einführungsstelle.

Es bestand daher der Bedarf nach einem kontinuierlichen Verfahren zur Herstellung von niederen Dialkylcarbonaten als Hauptprodukt und Alkylenglykol als Nebenprodukt durch katalysierte Umesterung eines zyklischen Alkylencarbonats mit niederen Alkoholen, bei dem die Mengen an zyklischem Alkylencarbonat möglichst gering sind.

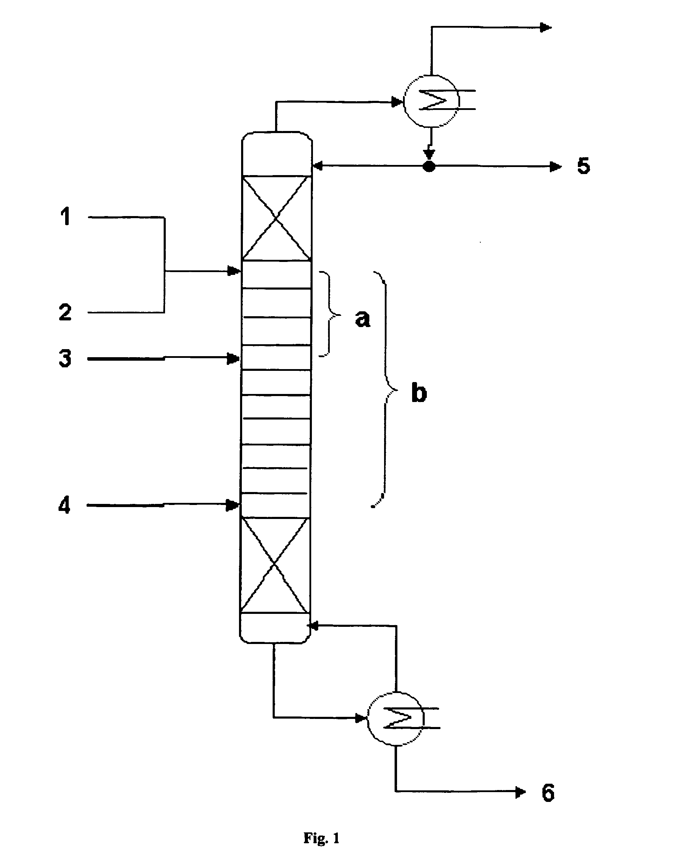

Die Aufgabe wurde überraschenderweise dadurch gelöst, dass das Verhältnis zwischen dem Abstand von Alkylencarbonat-Einführungsstelle bis zur ersten Alkohol-Einführungsstelle (siehe Fig. 1, Abstand a) und dem Abstand von Alkylencarbonat-Einführungsstelle bis zur zweiten Alkohol-Einführungsstelle (Fig. 1, Abstand b) von 0,2 bis 0,52, bevorzugt von 0,25 bis 0,48, besonders bevorzugt 0,28 bis 0,44 beträgt.

Im erfindungsgemäßen Verfahren wird für die erste Alkohol-Einführungsstelle ein Dialkylcarbonat enthaltender Alkohol, dessen Dialkylcarbonatgehalt bevorzugt von 0,2 bis 30 Gew.-% beträgt, eingesetzt.

Gegenstand der Erfindung ist also ein Verfahren zur kontinuierlichen Herstellung von Dialkylcarbonat der Formel (I)

(R¹O)₂CO (I),

in der R¹ geradkettiges oder verzweigtes C₁-C₄ darstellt,
als auch von Alkylenglykol als Nebenprodukt der Formel (II)

R²(OH)₂ (II)

in der R² ein C₂-C₄-Alkyl darstellt,
durch Umesterung eines zyklischen Alkylencarbonates mit einem Alkohol der Formel (III)

R¹OH (III),

in der R¹ die obige Bedeutung hat, in Gegenwart eines Katalysators
dadurch gekennzeichnet, dass die Umesterung in einer Kolonne im Gegenstrom so geführt wird, dass das zyklische Alkylencarbonat (1) in den oberen Teil der Kolonne und ein Dialkylcarbonat enthaltender Alkohol (3), dessen Dialkylcarbonatgehalt bevorzugt bei 0,2 bis 30 Gew.-% liegt, in den mittleren oder unteren Teil der Kolonne eingeführt werden und zusätzlich unterhalb der Einführung des Dialkylcarbonat enthaltenden Alkohols eine weitere Einführungsstelle für einen Alkohol enthaltenden Strom (4) vorgesehen wird,
wobei das Verhältnis zwischen dem Abstand von Alkylencarbonat-Einführungsstelle (1) bis zur Einführungsstelle des Dialkylcarbonat enthaltenden Alkohols (3) und dem Abstand von Alkylencarbonat-Einführungsstelle (1) bis zur zweiten Alkohol-Einführungsstelle (4) von 0,20 bis 0,52 liegt.

Überraschenderweise ist es im erfindungsgemäßen Verfahren durch die Einhaltung des genannten Abstandverhältnisses nicht mehr notwendig, reinen Alkohol in der zweiten, untersten Alkohol-Einführungsstelle einzusetzen. Hierbei reicht der Einsatz eines mindestens zu 90 Gew.-%, bevorzugt mindestens zu 95 Gew.-%, und besonders bevorzugt mindestens zu 99,5 Gew.-% reinen Alkohols völlig aus. Bevorzugt wird eine Reinheit des Alkohols von bis zu 99,99 Gew.-% verwendet.

Bevorzugt werden Gewichtsanteile an zyklischem Alkylencarbonat kleiner als 1000 ppm, besonders bevorzugt kleiner als 500 ppm, im Schwersiedergemisch, das im Kolonnensumpf kontinuierlich abgezogen wird.

Im Rahmen der Erfindung hergestellte Dialkylcarbonate sind bevorzugt solche der allgemeinen Formel (IV) 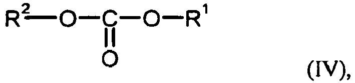 wobei R¹ und R² unabhängig voneinander für lineares oder verzweigtes, gegebenenfalls substituiertes C₁-C₃₄-Alkyl, bevorzugt für C₁-C₆-Alkyl, besonders bevorzugt für C₁-C₄-Alkyl stehen. Dabei können R¹ und R² gleich oder verschieden sein. Bevorzugt sind R¹ und R² gleich.

C₁-C₄-Alkyl steht im Rahmen der Erfindung beispielsweise für Methyl, Ethyl, n-Propyl, iso-Propyl, n-Butyl, sec.-Butyl, tert.-Butyl, C₁-C₆-Alkyl darüber hinaus beispielsweise für n-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl oder 1-Ethyl-2-methylpropyl, **C₁-C₃₄-Alkyl** darüber hinaus beispielsweise für n-Heptyl und n-Octyl, Pinakyl, Adamantyl, die isomeren Menthyle, n-Nonyl, n-Decyl, n-Dodecyl, n-Tridecyl, n-Tetradecyl, n-Hexadecyl oder n-Octadecyl. Gleiches gilt für den entsprechenden Alkylrest beispielsweise in Aralkyl- bzw. Alkylarylresten. Alkylenreste in den entsprechenden Hydroxyalkyl- oder Aralkyl- bzw. Alkylarylresten stehen beispielsweise für die den vorangehenden Alkylresten entsprechenden Alkylenreste.

Die vorangehenden Aufzählungen sind beispielhaft und nicht als Limitierung zu verstehen.

Bevorzugte Dialkylcarbonate sind Dimethylcarbonat, Diethylcarbonat, Di(n-propyl)carbonat, Di(iso-propyl)carbonat, Di(n-butyl)carbonat, Di(sec-butyl)carbonat, Di(tert-butyl)carbonat oder Dihexylcarbonat. Besonders bevorzugt sind Dimethylcarbonat oder Diethylcarbonat. Ganz besonders bevorzugt ist Dimethylcarbonat.

Im Rahmen der Erfindung verwendete zyklische Alkylencarbonate sind bevorzugt solche mit der Formel (V): 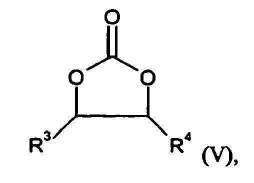 wobei in der Formel R³ und R⁴ unabhängig voneinander Wasserstoff, substituiertes oder nicht substituiertes **C₁-C₄-Alkyl,** substituiertes oder nicht substituiertes **C₂-C₄-Alkenyl** oder substituiertes oder nicht substituiertes **C₆-C₁₂-Aryl** und R³ und R⁴ gemeinsam mit den beiden Dreiring-C-Atomen einen gesättigten carbozyklischen Ring mit 5 - 8 Ringgliedern bedeuten können.

Die zyklischen Alkylencarbonate werden mit Alkoholen der Form

R⁵-OH

umgesetzt, wobei R⁵ ein geradkettiges oder verzweigtes **C₁-C₄-Alkyl** bedeutet.

Die erfindungsgemäß einsetzbaren Umesterungskatalysatoren sind die dem Fachmann bekannten, beispielsweise Hydride, Oxide, Hydroxide, Alkoholate, Amide oder Salze von Alkalimetallen, wie Lithium, Natrium, Kalium, Rubidium und Cäsium, bevorzugt von Lithium, Natrium und Kalium, besonders bevorzugt von Natrium und Kalium (US 3,642,858 A, US 3 803 201 A, EP 1 082 A). Für den Fall des Einsatzes der Alkoholate können diese erfindungsgemäß auch in situ durch Einsatz der elementaren Alkalimetalle und dem erfindungsgemäßen umzusetzenden Alkohol gebildet werden. Salze der Alkalimetalle können solche von organischen oder anorganischen Säuren sein, wie von Essigsäure, Propionsäure, Buttersäure, Benzoesäure, Stearinsäure, Kohlensäure (Carbonate oder Hydrogencarbonate), von Salzsäure, Bromwasserstoff oder lodwasserstoffsäure, Salpetersäure, Schwefelsäure, Fluorwasserstoffsäure, Phosphorsäure, Blausäure, Rhodanwasserstoff, Borsäure, Zinnsäure, C₁-C₄-Stannonsäuren oder Antimonsäuren. In bevorzugter Weise kommen als Verbindungen der Alkalimetalle die Oxide, Hydroxide, Alkoholate, Acetate, Propionate, Benzoate, Carbonate und Hydrogencarbonate in Frage, in besonders bevorzugter Weise werden Hydroxide, Alkoholate, Acetate, Benzoate oder Carbonate eingesetzt. Solche Alkalimetallverbindungen (gegebenenfalls in situ gebildet aus den freien Alkalimetallen) werden in Mengen von 0,001 bis 2 Gew.-%, bevorzugt 0,003 bis 1,0 Gew.-%, besonders bevorzugt 0,005 bis 1,0 Gew.-%, bezogen auf das umzusetzende Reaktionsgemisch, eingesetzt.

Es ist erfindungsgemäß möglich, solchen Alkalimetallverbindungen gegebenenfalls komplexierende Stoffe zuzusetzen. Beispielsweise seien genannt Kronenether wie Dibenzo-18-krone-6, Polyethylenglykole oder bicyclische stickstoffhaltige Kryptanden.

Solche Komplexiermittel werden in Mengen von 0,1 bis 200 mol-%, bevorzugt in 1 bis 100 mol-%, bezogen auf die Alkalimetallverbindung, eingesetzt.

Als Katalysatoren für das erfindungsgemäße Verfahren sind ferner Thallium-I- und Thallium-111-Verbindungen geeignet, wie die Oxide, Hydroxide, Carbonate, Acetate, Bromide, Chloride, Fluoride, Formiate, Nitrate, Cyanate, Stearate, Naphthenate, Benzoate, Cyclohexylphosphonate, Hexahydrobenzoate, das Cyclopentadienylthallium, Thalliummethylat, Thalliumethylat, bevorzugt TI-(I)-oxid, TI-(I)-hydroxid, TI-(I)-carbonat, TI-(I)-acetat, TI-(111)-acetat, TI-(I)-fluorid, TI-(I)-forrniat, TI-(I)-nitrat, TI-(I)-naphtenat und TI-(I)-methylat (EP 1 083). Die Mengen an Thalliumkatalysator sind nicht besonders kritisch. Sie betragen im allgemeinen 0,0001-10 Gew.-%, bevorzugt 0,001-1 Gew.-%, bezogen auf das gesamte Reaktionsgemisch. Im erfindungsgemäßen Verfahren können weiterhin stickstoffhaltige Basen als Katalysatoren eingesetzt werden (US 4 062 884). Genannt seien beispielsweise sek. oder tert. Amine wie Triethylamin, Tributylamin, Methyldibenzylamin, Dimethylcyclohexylamin u.a..

Die erfindungsgemäß eingesetzten Mengen der stickstoffhaltigen Basen liegen von 0,01 bis 10 Gew.-%, bevorzugt von 0,1 bis 5 Gew.-%, besonders bevorzugt von 0,1 bis 1 Gew.-%, bezogen auf das gesamte Reaktionsgemisch. Als Katalysatoren sind erfmdungsgemäß ferner Verbindungen aus der Gruppe der Phosphine, Stibine, Arsine oder der zweiwertigen Schwefel- und Selen-Verbindungen sowie deren Oniumsalze einsetzbar (EP 180 387, US 4 734 519).

Beispielhaft seien die folgenden genannt: Tributylphosphin, Triphenylphosphin, Diphenylphosphin, 1,3-Bis-(diphenylphosphino)propane, Triphenylarsin, Trimethylarsin, Tributylarsin, 1,2-Bis-(diphenyl-arsino)ethan, Triphenylantimon, Diphenylsulfid, Diphenyldisulfid, Diphenylselenid, Tetraphenylphosphoniumhalogenid (CI, Br, J), Tetraphenylarsoniumhalogenid (CI, Br, J), Triphenylsulfoniumhalogenid (C1, Br) usw.

Die erfindungsgemäßen Einsatzmengen dieser Katalysatorengruppe liegen im Bereich von 0,1 bis 10 Gew.-%, bevorzugt von 0,1 bis 5 Gew.-%, besonders bevorzugt im Bereich von 0,1 bis 2 Gew.-%, bezogen auf das gesamte Reaktionsgemisch.

Weiterhin erfindungsgemäß einsetzbar sind Komplexe oder Salze des Zinns, Titans oder Zirkoniums (US 4 661 609). Beispiele solcher Systeme sind Butylstannonsäure, Zinnmethoxid, Dimethylzinn, Dibutylzinnoxid, Dibutylzinndilaurat, Tributylzinnhydrid, Tributylzinnchlorid, Zinn(11)ethylhexanoate, Zirkoniumalkoxide (Methyl, Ethyl, Butyl), Zirkonium(1V)halogenide (F, CI, Br, J), Zirkoniumnitrate, Zirkoniumacetylacetonat, Titanalkoxide (Methyl, Ethyl, Isopropyl), Titanacetat, Titanacetylacetonat usw.

Die erfindungsgemäß einsetzbaren Mengen betragen 0,1 bis 10 Gew.-%, bevorzugt 0,1 bis 5 Gew.-%, bezogen auf das Gesamtgemisch.

Im erfindungsgemäßen Verfahren können weiterhin bifunktionelle Katalysatoren der Formel (VI)

[AₐX_{b}]ₘ• [B_{c}Y_{d}]ₙ (VI)

eingesetzt werden. In diesen bifunktionellen Katalysatoren wird das molare Verhältnis der beiden in eckigen Klammern stehenden Komponenten durch die Indices m und n ausgedrückt. Diese Indices können unabhängig voneinander Werte von 0,001-1, bevorzugt 0,01-1, besonders bevorzugt 0,05-1 und ganz besonders bevorzugt 0,1-1 annehmen. Innerhalb der eckigen Klammern stehen neutrale Salze aus je einem Kation und einem Anion. Die Indices a und b stellen unabhängig voneinander ganze Zahlen von 1-5 dar; die Indices C und d bedeuten unabhängig voneinander ganze Zahlen von 1-3, wobei den Forderungen der Valenzen der Kationen und Anionen zur Bildung solcher neutraler Salze zu entsprechen ist. Des weiteren bedeuten in (VI) A das Kation eines Metalles, das der dritten Periode und Gruppe IIa, der vierten Periode und Gruppe IIa, IVa-VIIIa, Ib oder IIb, der fünften Periode und Gruppe IIa, IVa-VIIa oder IVb bzw. der sechsten Periode und Gruppe IIa-VIa des Periodensystems der Elemente in der Kurzperiodenform angehört.

Die in Frage kommenden Metalle für das Kation A entnimmt der Fachmann den üblichen Darstellungen des Periodensystems der Elemente (Mendelejew) in der Kurzperiodenform. In bevorzugter Weise handelt es sich bei A um das Kation eines der Metalle Mg, Ca, Sr, Ba, Zn, Cu, Mn, Co, Ni, Fe, Cr, Mo, W, Ti, Zr, Sn, Hf, V und Ta, in bevorzugter Weise um das Kation eines der Metalle Mg, Ca, Zn, Co, Ni, Mn, Cu und Sn. Außer den nicht komplexierten Kationen der genannten Metalle kommen auch kationische Oxokomplexe der genannten Metalle in Frage, wie beispielsweise Titanyl TiO⁺⁺ und Chromyl CrO₂⁺⁺.

Das zum Kation A gehörige Anion X ist das einer anorganischen oder organischen Säure. Eine solche anorganische oder organische Säure kann einbasisch oder zweibasisch oder dreibasisch sein. Solche Säuren und ihre Anionen sind dem Fachmann bekannt. Beispiele für Anionen einbasischer anorganischer oder organischer Säuren sind: Fluorid, Bromid, Chlorid, lodid, Nitrat, das Anion einer Alkancarbonsäure mit 1-18 C-Atomen und Benzoat; Beispiele für Anionen zweibasischer anorganischer oder organischer Säuren sind: Sulfat, Oxalat, Succinat, Fumarat, Maleinat, Phthalat und weitere; Beispiele für dreibasische anorganische oder organische Anionen sind: Phosphat oder Citrat. Bevorzugte Anionen X im Katalysator der Formel (VI) sind: Fluorid, Chlorid, Bromid, lodid, Sulfat, Nitrat, Phosphat, Formiat, Acetat, Propionat, Oxalat, Butyrat, Citrat, Succinat, Fumarat, Maleinat, Benzoat, Phthalat, Decanoat, Stearat, Palmitat, und Laurinat. Besonders bevorzugte Anionen X sind: Chlorid, Bromid, lodid, Acetat, Laurinat, Stearat, Palmitat, Decanoat, Nitrat und Sulfat.

Als Kation B in den Katalysatoren der Formel (VI) kommt eines aus der Gruppe der Alkali- oder Erdalkalikationen, der quaternären Ammonium-, Phosphonium-, Arsonium- oder Stibonium-Kationen und der ternären Sulfonium-Kationen in Frage.

Als (Erd)Alkalimetallkationen seien hierbei genannt: das Lithium-, Natrium-, Kalium-, Rubidium-, Cäsium-, Magnesium-, Calcium-, Strontium- und Bariumkation, bevorzugt die genannten Alkalimetallkationen, besonders bevorzugt das Natrium- und das Kaliumkation.

In bevorzugter Weise kommen als Kationen B solche der Formel (VII) 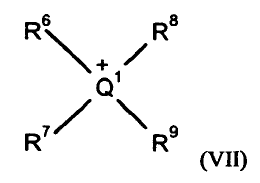 in Frage, worin
Q¹ für N, P, As oder Sb steht und
R⁶, R⁷, R⁸ und R⁹ unabhängig voneinander geradkettiges oder verzweigtes C₁-C₁₈ oder C₇-C₁₂-Aralkyl sind und einer der Reste R⁶-R⁹ auch sein kann. In besonders bevorzugter Weise ist B ein Kation der Formel (VIII) 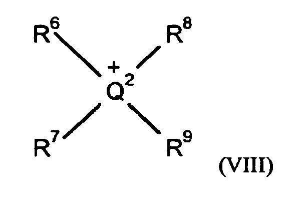 worin,
Q² für N oder P, bevorzugt für N steht.

In ganz besonders bevorzugter Weise treten im Rahmen der Formeln (VII) bzw. (VIII) an die Stelle der Reste R⁶, R⁷, R⁸ und R⁹ die Reste R¹⁶, R¹⁷, R¹⁸ bzw. R¹⁹, die unabhängig voneinander geradkettiges oder verzweigtes C₁-C₁₈-Alkyl oder C₇-C₈-Aralkyl bedeuten und einer der Reste R¹⁶ bis R¹⁹ auch Phenyl sein kann. In weiterhin ganz besonders bevorzugter Weise treten an die Stelle der Reste R¹⁶, R¹⁷, R¹⁸ bzw. R¹⁹, die Reste R²⁶, R²⁷, R²⁸ bzw. R²⁹, die unabhängig voneinander C₁-C₈-Alkyl oder Benzyl bedeuten und einer der Reste R²⁶ bis R²⁹ auch Phenyl sein kann.

Geradkettiges oder verzweigtes C₁-C₁₈Alkyl ist beispielsweise Methyl, Ethyl, Propyl, Isopropyl, Butyl, Isobutyl, Hexyl, Octyl, Dodecyl, Hexadecyl oder Octadecyl. Bevorzugtes Alkyl hat 1-12 C-Atome, besonders bevorzugtes Alkyl hat 1-8 C-Atome.

C₇-C₁₂-Aralkyl ist beispielsweise Benzyl, Phenylethyl, Phenylpropyl, Naphthylmethyl oder Naphthylethyl; bevorzugtes Aralkyl ist Benzyl oder Phenylethyl, ganz besonders bevorzugtes Aralkyl ist Benzyl.

C₆-C₁₂-Aryl ist beispielsweise Phenyl, Naphthyl oder Biphenylyl, bevorzugt Phenyl.

Das Anion Y im Katalysator der Formel (VI) ist ein Halogenidion, wie Fluorid, Chlorid, Bromid oder lodid, bevorzugt Bromid oder lodid, besonders bevorzugt Iodid. Es kann jedoch auch die Bedeutung von anderen unter X genannten Anionen besitzen, wenn im konkreten Fall das Anion X Bromid oder lodid ist.

Der bifunktionelle Katalysator der Formel (VI) wird in einer Menge von 0,005-5 Gew.-%, bevorzugt 0,01-3 Gew.-%, besonders bevorzugt 0,01-1 Gew.-%, bezogen auf das gesamte Umesterungsgemisch, eingesetzt.

Diese Katalysatormengen unterscheiden sich teilweise von den in der Literatur genannten Mengen. Besonders überraschend ist, dass im erfindungsgemäßen Verfahren relativ hohe Konzentrationen an den wirksamen Katalysatoren auf der Basis von Alkaliverbindungen eingesetzt werden können, ohne dass es dabei zu den ausbeutemindernden und die Reaktionsführung behindernden Entwicklungen von CO₂ und zur Bildung von Polyolen kommt, wie dies beispielsweise aus DE-OS 2 740 243 und der dort zitierten Literatur und aus DE-OS 2 740 251 bekannt ist. Auch dies ist eine überraschende Besonderheit des erfindungsgemäßen Verfahrens.

Solche Katalysatoren können homogen gelöst auf den Kopf der Kolonne gegeben werden, wobei als Lösungsmittel Alkylencarbonat, Alkylenglykol, Alkohol oder Dialkylcarbonat, also systemeigene Lösungsmittel, zur Anwendung gelangen. Es ist selbstverständlich möglich, nicht lösliche Umesterungskatalysatoren einzusetzen, die auf den Zwischenböden oder inmitten der Füllkörper angeordnet sind. In einem solchen Fall kann die Dosierung eines gelösten Katalysators über (2) entfallen. Geeignete heterogene Katalysatoren sind beispielsweise:
lonentauscherharze mit funktionellen Gruppen aus tert. Aminen, quartären Ammoniumgruppen, wobei als Gegenionen Hydroxid, Chlorid oder Hydrogensulfat beispielsweise genannt seien, Sulfonsäuregruppen oder Carboxylgruppen, wobei für beide als Gegenionen Wasserstoff, Alkalimetalle oder Erdalkalimetalle beispielhaft genannt seien. Diese funktionellen Gruppen können entweder direkt oder über inerte Ketten an das Polymer gebunden sein (US 4,062,884 A, US 4,691,041 A, EP 298 167 A). Weiterhin genannt seien Alkali- oder Erdalkalisilikate, imprägniert auf Siliciumdioxidträgern, sowie Ammonium-ausgetauschte Zeolithe.

Das erfindungsgemäße Verfahren kann kontinuierlich oder diskontinuierlich durchgeführt werden. Bevorzugt ist eine kontinuierliche Fahrweise.

Im erfindungsgemäßen Verfahren werden die zyklische(n) Alkylencarbonatverbindung(en) und der oder die Alkohol(e) bevorzugt im Molverhältnis von 1 : 0.1 bis 1 : 40, besonders bevorzugt von 1 : 1.0 bis 1 : 30, ganz besonders bevorzugt von 1 : 2.0 bis 1 : 20 eingesetzt. Dabei berücksichtigt das angegebene Molverhältnis nicht die Rückführung von zyklischer Alkylencarbonatverbindung oder Alkohol in die Umesterungskolonne über einen oder mehrere Kopfkondensator(en) (vgl. unter (b)) oder einen oder mehrere etwaig vorhandene(n) Sumpfverdampfer.

Der Katalysator wird bevorzugt zusammen mit dem Strom enthaltend das zyklische Alkylencarbonat in gelöster oder suspendierter Form in die Umesterungskolonne über eine Einführungsstelle, die oberhalb der Einführungsstellen des Alkohols angeordnet ist, in die Kolonne eingebracht. Alternativ kann der Katalysator auch separat beispielsweise im Alkohol, im Alkylenglykol oder in einem geeigneten inerten Lösungsmittel gelöst, zudosiert werden. Im Falle der Verwendung heterogener Katalysatoren können diese im Gemisch mit den genannten Füllkörpern, in geeigneter Form anstelle von Füllkörpern oder als Schüttung auf etwaig eingebaute Kolonnenböden eingesetzt werden.

Das erfindungsgemäße Verfahren wird in einer Umesterungskolonne durchgeführt. In bevorzugten Ausführungsformen des erfindungsgemäßen Verfahrens kann der am Sumpf dieser Umesterungskolonne entnommene Flüssigkeitsstrom - gegebenenfalls nach Aufkonzentrierung - in einem oder mehreren weiteren Schritten einer weiteren Reaktion und/oder Reinigung unterzogen werden. Bevorzugt können einzelne oder alle solche weiteren Schritte in einer oder mehreren weiteren Kolonnen erfolgen.

Als Umesterungskolonne oder gegebenenfalls zweite bzw. weitere Kolonne(n) kommen dem Fachmann bekannte Kolonnen in Frage. Dies sind beispielsweise Destillations- bzw. Rektifikationskolonnen, bevorzugt Reaktivdestillations- bzw. Reaktivrektifikationskolonnen.

Die Umesterungskolonne enthält bevorzugt wenigstens einen Verstärkungsteil im oberen Teil der Kolonne und wenigstens eine Reaktionszone unterhalb des Verstärkungsteils. Jede der zwei Sektionen weist unabhängig voneinander bevorzugt jeweils 0 bis 30, bevorzugt 0,1 bis 30 theoretische Stufen auf In bevorzugten Ausführungsformen weist die Umesterungskolonne unterhalb einer Reaktionszone wenigstens einen Abtriebsteil auf.

Die Umesterungskolonne kann weiterhin bevorzugt mit einem oder mehreren Sumpfverdampfer(n) ausgestattet sein. Bei Ausführung der Umesterungskolonne mit einem Abtriebsteil wird bevorzugt zusätzlich ein Sumpfverdampfer eingesetzt, welcher die aus dem Abtriebsteil ablaufende Flüssigkeit ganz oder teilweise verdampft. Dieser ganz oder teilweise verdampfte Flüssigkeitsstrom wird ganz oder teilweise wieder in die Umesterungskolonne zurückgeführt. Im Falle einer Ausführungsform ohne Abtriebsteil wird in einem gegebenenfalls eingesetzten Sumpfverdampfer die aus der Reaktionszone ablaufende Flüssigkeit ganz oder teilweise verdampft und ganz oder teilweise wieder in die Umesterungskolonne zurückgeführt.

Der oder die Verstärkungsteil(e) können in bevorzugten Ausführungsformen zusammen mit dem oder den Reaktionsteil(en) und gegebenenfalls wenigstens einem Abtriebsteil in der Umesterungskolonne untergebracht werden. Dabei wird das aus der oder den Reaktionszone(n) kommende dampfförmige Gemisch von unten in eine untere Sektion des Verstärkungsteils bzw. gegebenenfalls den unteren Verstärkungsteil geleitet, wobei eine Abreicherung des Alkylencarbonats bzw. Alkylenglykols stattfindet.

Unterhalb der Reaktionszone und eines gegebenenfalls vorhandenen Abtriebsteils wird ein Gemisch enthaltend Alkylenglykol, überschüssigem oder nichtumgesetztem Alkylencarbonat, Alkohol, Dialkylcarbonat, Umesterungskatalysatoren und bei der Reaktion entstehende oder bereits in den Edukten enthaltene schwersiedende Verbindungen erhalten. Bei Verwendung eines Abtriebsteils wird der Gehalt an leichtsiedenden Verbindungen, wie beispielsweise Dialkylcarbonat und Alkohol reduziert, wobei in Anwesenheit des Umesterungskatalysators unter Umständen weiteres Dialkylcarbonat und Alkylenglykol gebildet werden. Die hierzu erforderliche Energie, wird bevorzugt durch einen oder mehrere Verdampfer zugeführt.

In allen Abschnitten der Umesterungskolonne, d.h. sowohl in Verstärkungs- und gegebenenfalls Abtriebsteil als auch in der Reaktionszone können Füllkörper oder geordnete Packungen zum Einsatz kommen. Die zu verwendenden Füllkörper bzw. geordneten Packungen sind die für Destillationen üblichen, wie sie beispielsweise in Ullmann's Encyclopädie der Technischen Chemie, 4. Aufl" Bd. 2, S. 528 ff. beschrieben sind. Als Beispiele für Füllkörper seien Raschig- oder Pall- und Novaloxringe, Berl-, Intalex- oder Torussättel, Interpackkörper und als Beispiele für geordnete Packungen seien Blech und Gewebepackungen (wie z.B. BX-Packungen, Montz Pak, Mellapak, Melladur, Kerapak und CY-Packung) aus verschiedenen Materialien, wie Glas, Steinzeug, Porzellan, Edelstahl, Kunststoff genannt. Bevorzugt sind Füllkörper und geordnete Packungen, die eine große Oberfläche, eine gute Benetzung sowie ausreichende Verweilzeit der Flüssigphase aufweisen. Diese sind beispielsweise Pall- und Novolaxringe, Berlsättel, BX-Packungen, Montz Pak, Mellapak, Melladur, Kerapak und CY-Packungen.

Alternativ eignen sich auch Kolonnenböden, wie beispielesweise Sieb-, Glocken-, Ventil-, Tunnelböden. In der oder den Reaktionszone(n) der Umesterungskolonne sind Kolonnenböden mit hohen Verweilzeiten bei gutem Stoffaustausch, beispielsweise Glockenböden, Ventil- oder Tunnelböden mit hohen Überlaufwehren, besonders bevorzugt. Die theoretische Bodenzahl der Reaktionszone beträgt vorzugsweise 3 bis 50, besonders bevorzugt 10 bis 50 und ganz besonders bevorzugt 10 bis 40. Der Flüssigkeits-Hold-up beträgt vorzugsweise 1 bis 80%, besonders bevorzugt 5 bis 70% und ganz besonders bevorzugt 7 bis 60% des Kolonneninnenvolumens der Reaktionszone. Die genauere Auslegung der Reaktionszone(n), des gegebenenfalls zu verwendendes Abtriebsteils und des oder der Verstärkerteil(e) kann vom Fachmann vorgenommen werden.

Die Temperatur der Reaktionszone(n) liegt bevorzugt im Bereich von 20 bis 200°C, besonders bevorzugt von 40 bis 180°C, ganz besonders bevorzugt von 50 bis 160°C. Es ist von Vorteil, die erfindungsgemäße Umesterung nicht nur bei Normaldruck, sondern auch bei erhöhtem oder erniedrigtem Druck durchzuführen. Der Druck der Reaktionszone liegt daher bevorzugt im Bereich von 0,2 bis 20 bar, besonders bevorzugt von 0,3 bis 10 bar, ganz besonders bevorzugt von 0,4 bis 5 bar. Bei den vorangehend und im Folgenden aufgeführten Druckangaben handelt es sich - sofern nichts anderes explizit erwähnt - um abolute Druckangaben.

Das erfindungsgemäße Verfahren wird ausschnittsweise mit Hilfe der Fig. 1 beispielhaft erläutert. Darin bedeuten:

| | |
|---|---|
| 1: | Strom, enthaltend zyklisches Alkylencarbonat |
| 2: | Strom, enthaltend den Katalysator als Suspension oder in einem systemeigenen Stoff gelöst (im Falle, dass in dem Verfahren ein homogener Katalysator verwendet wird); systemeigene Stoffe sind: Alkylencarbonat, Alkohol, Dialkylcarbonat oder Alkylenglykol |
| 3: | Strom, enthaltend Alkohol und in geringeren Anteilen Dialkylcarbonat |
| 4: | Strom, enthaltend Alkohol |
| 5: | Leichtsieder-Strom, enthaltend u.a. Dialkylcarbonat und Alkohol |
| 6: | Hochsieder-Strom, enthaltend u.a. Alkylenglykol, Alkylencarbonat, Alkohol, Hochsieder und Spuren weiterer Nebenprodukte und ggf. Katalysator |
| a: | Abstand zwischen Einführungsstelle des Alkylencarbonats und Strom 3 |
| b: | Abstand zwischen Einführungsstelle des Alkylencarbonats und Strom 4 |

Fig. 1 zeigt eine Umesterungskolonne, in die die drei Eduktströme, d.h. ein das zyklische Alkylencarbonat enthaltender Strom 1, der Alkohol und in kleineren Mengen das Dialkylcarbonat enthaltende Strom 3 und der in hoher Konzentration Alkohol enthaltende Strom 4 im Bereich einer Reaktionszone RZ im Sinne eine Gegenstromumesterung gegeneinander geführt und zu Dialkylcarbonaten und Alkylenglykolen umgesetzt werden.

Die Ströme 3 und 4 werden im Gegenstrom zu Strom 1 geführt. Strom 1 wird der Umesterungskolonne flüssig zugeführt, während die Ströme 3 und 4 gasförmig und ggf. leicht überhitzt in die Kolonne geführt werden. Je nach Quelle der verwendeten Edukte enthalten diese entsprechend typische Verunreinigungen. Der Strom 3 enthält als Hauptkomponente Alkohol und beispielsweise 0 bis 40 Gew. %, bevorzugt 0,1 - 35 Gew. % und besonders bevorzugt 0,2 - 30 Gew. % des Dialkylcarbonats. Weitere Komponenten des Stromes 3 haben in Summe < 1 Gew.-% Anteil. Strom 4 hat demgegenüber einen Anteil von > 90 Gew. %, bevorzugt > 95 Gew. % und besonders bevorzugt > 99 Gew. % Alkohol.

Das molare Verhältnis des über Strom 1 der Kolonne zugeführten Alkylencarbonats zur Gesamtmenge des über die Ströme 3 und 4 zugeführten Alkohols beträgt 1 : 0,1 bis 1 : 40, besonders bevorzugt von 1 : 1,0 bis 1 : 30, ganz besonders bevorzugt von 1 : 2,0 bis 1 : 20. Die Gesamtmenge des über die Ströme 3 und 4 zugeführten Alkohols ist im Verhältnis auf den Strom 4 zum Strom 3 aufgeteilt wie 1 : 1 bis 1 : 15, bevorzugt 1 : 1,2 bis 1 : 12, besonders bevorzugt 1 : 1,4 bis 1 : 8.

Das molare Verhältnis des über Strom 2 der Kolonne zugeführten Katalysators zu Strom 1 des Alkylencarbonats beträgt 0,01 bis 2 mol%, bevorzugt 0,02 bis 1,8 mol%, besonders bevorzugt 0,03 bis 1,6 mol%.

Die Kolonne in Fig. 1 besteht aus einem über der Reaktionszone liegenden Verstärkungsteil, der Reaktionszone selbst und dem unterhalb der Reaktionszone liegenden Abtriebsteil. Die - geometrische - Definition der Reaktionszone ist durch 2 Elemente festgelegt, der zuoberst angeordneten Zuführung des Alkylencarbonats (Strom 1) und der zuunterst angeordneten Zuführung des Alkohols (Strom 4). Die Reaktion von Alkylencarbonat und Alkohol zu Dialkylcarbonat und Alkylenglykol ist eine zweistufige Gleichgewichtsreaktion (s. z.B. US 6930195 B2). Sowohl die Hin- als auch die Rückreaktionen sind nicht auf die Reaktionszone beschränkt.

Die folgenden Beispiele dienen der exemplarischen Erläuterung der Erfindung und sind nicht als Beschränkung aufzufassen.

### Beispiele

### Beispiel 1:

Eine reaktive Destillationskolonne bestehend aus einem Verstärkungsteil mit 9 theoretischen Stufen, einer Reaktionszone mit 25 Reaktionsböden (HoldupBoden: 0,6 m³) und einem Abtriebsteil mit 4 theoretischen Stufen wird bei einem am Kopf der Kolonne gemessenen Druck von 400 mbar (absolut) und einem Rücklaufverhältnis von 0,66 betrieben.

In den oberen Kolonnenbereich direkt oberhalb des ersten Reaktionsbodens werden kontinuierlich 9000 kg/h Ethylencarbonat und 58 kg/h eines Gemisches mit 33,3 Gew.-% KOH und 66,7 Gew.-% Ethylenglykol zudosiert. Zwischen dem 8. und 9. Reaktionsboden werden 21437 kg/h eines Dampfgemisches mit 83,7 Gew.-% Methanol und 16,3 Gew.-% Dimethylcarbonat zugeführt. Zusätzlich dazu werden am unteren Ende der Reaktionszone 7146 kg/h eines Dampfgemisches mit 99,5 Gew.-% Methanol, 0,45 Gew.-% Ethylenglykol und 500 ppm Dimethylcarbonat zugeführt. Dies entspricht ein Abstandverhältnis a/b von 0,36.

Ein Partialkondensator kondensiert den Dampfstrom am Kopf der Kolonne bei 40°C. Man erhält sowohl 6 kg/h dampfförmiges als auch 30729 kg/h flüssiges Destillat mit einer Zusammensetzung von 59 Gew.-% Methanol und 41 Gew.-% Dimethylcarbonat, das weiteren Aufreinigungsschritten zugeführt wird.

Der Sumpfverdampfer wird bei 102°C betrieben und man erhält 7022 kg/h flüssiges Sumpfprodukt, das hauptsächlich Ethylenglykol und unter anderem 400 ppm Ethylencarbonat beinhaltet.

### Vereleichsbeisniel 1 :

Es wird die gleiche reaktive Destillationskolonne, wie bereits im Beispiel 1 beschrieben, verwendet.

Die Kolonne wird bei einem am Kopf der Kolonne gemessenen Druck von 400 mbar (absolut) und einem Rücklaufverhältnis von 0,66 betrieben.

In den oberen Kolonnenbereich direkt oberhalb des ersten Reaktionsbodens werden kontinuierlich 9000 kg/h Ethylencarbonat und 58 kg/h eines Gemisches mit 33,3 Gew.-% KOH und 66,7 Gew.-% Ethylenglykol zudosiert. Zwischen dem 14. und 15. Reaktionsboden werden 21437 kg/h eines Dampfgemisches mit 83,7 Gew.-% Methanol und 16,3 Gew.-% Dimethylcarbonat zugeführt. Zusätzlich dazu werden am unteren Ende der Reaktionszone 7146 kg/h eines Dampfgemisches mit 99,5 Gew.-% Methanol, 0,45 Gew.-% Ethylenglykol und 500 ppm Dimethylcarbonat zugeführt. Dies entspricht ein Abstandverhältnis a/b von 0,6. Das Mengenverhältnis zwischen dem insgesamt zugeführten Methanol und dem Ethylencarbonat bleibt gegenüber Beispiel 1 konstant.

Ein Partialkondensator kondensiert den Dampfstrom am Kopf der Kolonne bei 40°C. Man erhält sowohl 6 kg/h dampfförmiges als auch 30727 kg/h flüssiges Destillat mit einer Zusammensetzung von 59 Gew.-% Methanol und 41 Gew.-% Dimethylcarbonat, das weiteren Aufreinigungsschritten zugeführt wird.

Der Sumpfverdampfer wird bei 102°C betrieben und man erhält 7024 kg/h flüssiges Sumpfprodukt, das hauptsächlich Ethylenglykol und unter anderem 1100 ppm Ethylencarbonat beinhaltet.

### Vergleichsbeispiel 2:

Es wird die gleiche reaktive Destillationskolonne, wie bereits im Beispiel 1 beschrieben, verwendet.

Die Kolonne wird bei einem am Kopf der Kolonne gemessenen Druck von 400 mbar (absolut) und einem Rücklaufverhältnis von 0,66 betrieben.

In den oberen Kolonnenbereich direkt oberhalb des ersten Reaktionsbodens werden kontinuierlich 9000 kg/h Ethylencarbonat und 58 kg/h eines Gemisches mit 33,3 Gew.-% KOH und 66,7 Gew.-% Ethylenglykol zudosiert. Zwischen dem 10. und 11. Reaktionsboden werden 25830 kg/h eines Dampfgemisches mit 97 Gew.-% Methanol und 3 Gew.-% Dimethylcarbonat zugeführt. Es wurde auf weitere Einführungsstelle für Methanol verzichtet. Das Mengenverhältnis zwischen dem insgesamt zugeführten Methanol und dem Ethylencarbonat bleibt gegenüber Beispiel 1 konstant.

Ein Partialkondensator kondensiert den Dampfstrom am Kopf der Kolonne bei 40°C. Man erhält sowohl 6 kg/h dampfförmiges als auch 27998 kg/h flüssiges Destillat mit einer Zusammensetzung von 64 Gew.-% Methanol und 36 Gew.-% Dimethylcarbonat, das weiteren Aufreinigungsschritten zugeführt wird.

Der Sumpfverdampfer wird bei 102°C betrieben und man erhält 7002 kg/h flüssiges Sumpfprodukt, das hauptsächlich Ethylenglykol und unter anderem 5000 ppm Ethylencarbonat beinhaltet.

### Vergleichsbeispiel 3:

Es wird die gleiche reaktive Destillationskolonne, wie bereits im Beispiel 1 beschrieben, verwendet.

Die Kolonne wird bei einem am Kopf der Kolonne gemessenen Druck von 400 mbar (absolut) und einem Rücklaufverhältnis von 0,66 betrieben.

In den oberen Kolonnenbereich direkt oberhalb des ersten Reaktionsbodens werden kontinuierlich 9000 kg/h Ethylencarbonat und 58 kg/h eines Gemisches mit 33,3 Gew.-% KOH und 66,7 Gew.-% Ethylenglykol zudosiert. Zwischen dem 19. und 20. Reaktionsboden werden 29935 kg/h eines Dampfgemisches mit 83,7 Gew.-% Methanol und 16,3 Gew.-% Dimethylcarbonat zugeführt. Es wurde auf weitere Einführungsstelle für Methanol verzichtet. Das Mengenverhältnis zwischen dem insgesamt zugeführten Methanol und dem Ethylencarbonat bleibt gegenüber Beispiel 1 konstant.

Ein Partialkondensator kondensiert den Dampfstrom am Kopf der Kolonne bei 40°C. Man erhält sowohl 6 kg/h dampfförmiges als auch 31938 kg/h flüssiges Destillat mit einer Zusammensetzung von 57,4 Gew.-% Methanol und 42,6 Gew.-% Dimethylcarbonat, das weiteren Aufreinigungsschritten zugeführt wird.

Der Sumpfverdampfer wird bei 102°C betrieben und man erhält 7166 kg/h flüssiges Sumpfprodukt, das hauptsächlich Ethylenglykol und unter anderem 5,3 Gew.-% Ethylencarbonat beinhaltet.

## Patentansprüche

1. Verfahren zur kontinuierlichen Herstellung sowohl von Dialkylcarbonat der Formel (I)
(R¹O)₂CO (I),
in der R¹ geradkettiges oder verzweigtes C₁-C₄ darstellt,
als auch von Alkylenglykol als Nebenprodukt der Formel (II)
R²(OH)₂ (II)
in der R² ein C₂-C₄-Alkyl darstellt,
durch Umesterung eines zyklischen Alkylencarbonates mit einem Alkohol der Formel (III)
R¹OH (III),
in der R¹ die obige Bedeutung hat, in Gegenwart eines Katalysators
**dadurch gekennzeichnet, dass** die Umesterung in einer Kolonne im Gegenstrom so geführt wird, dass das zyklische Alkylencarbonat (1) in den oberen Teil der Kolonne und ein Dialkylcarbonat enthaltender Alkohol (3), in den mittleren oder
unteren Teil der Kolonne eingeführt werden und zusätzlich unterhalb der Einführung des Dialkylcarbonat enthaltenden Alkohols eine weitere Einführungsstelle für einen Alkohol enthaltenden Strom (4) vorgesehen wird,
wobei das Verhältnis zwischen dem Abstand von Alkylencarbonat-Einführungsstelle (1) bis zur Einführungsstelle des Dialkylcarbonat enthaltenden Alkohols (3) und dem Abstand von Alkylencarbonat-Einführungsstelle (1) bis zur zweiten Alkohol-Einführungsstelle (4) von 0,20 bis 0,52 liegt.

2. Verfahren nach Anspruch 1, **dadurch gekennzeichnet, dass** das Verhältnis zwischen dem Abstand von Alkylencarbonat-Einführungsstelle (1) bis zur Einführungsstelle des Dialkylcarbonat enthaltenden Alkohols (3) und dem Abstand von Alkylencarbonat-Einführungsstelle (1) bis zur zweiten Alkohol-Einführungsstelle (4) von 0,28 bis 0,44 liegt.

3. Verfahren nach einem der Ansprüche 1 oder 2, **dadurch gekennzeichnet, dass** im ein Dialkylcarbonat enthaltender Alkohol (3) der Dialkylcarbonatgehalt bevorzugt bei 0,2 bis 30 Gew.-% liegt.

4. Verfahren nach einem der Ansprüche 1 bis 3, **dadurch gekennzeichnet, dass** als zyklisches Alkylencarbonat Ethylen- oder Propylencarbonat verwendet wird.

5. Verfahren nach einem der Ansprüche 1 bis 4, **dadurch gekennzeichnet, dass** als Alkohol Methanol und als Dialkylcarbonat Dimethylcarbonat verwendet wird.

6. Verfahren nach einem der Ansprüche 1 bis 5, **dadurch gekennzeichnet, dass** in die zweite, unterste Alkohol-Einführungsstelle (4) mindestens zu 90 Gew.-%, bevorzugt mindestens zu 95 Gew.-%, und besonders bevorzugt mindestens zu 99,5 Gew.-% reiner Alkohol eingeführt wird.

7. Verfahren nach einem der Ansprüche 1 bis 6, **dadurch gekennzeichnet, dass** die Reaktion in Gegenwart eines homogenen Katalysators durchgeführt wird.

8. Verfahren nach einem der Ansprüche 1 bis 7, **dadurch gekennzeichnet, dass** als Katalysator Kalium- oder Natriumhydroxid verwendet wird.

9. Verfahren nach einem der Ansprüche 1 bis 8, **dadurch gekennzeichnet, dass** die Umesterungskolonne wenigstens einen Verstärkungsteil im oberen Teil der Kolonne und wenigstens eine Reaktionszone unterhalb des Verstärkungsteils enthält.

10. Verfahren nach einem der Ansprüche 1 bis 9, **dadurch gekennzeichnet, dass** die Umesterungskolonne unterhalb einer Reaktionszone wenigstens einen Abtriebsteil aufweist.

11. Verfahren nach einem der Ansprüche 1 bis 10, **dadurch gekennzeichnet, dass** die Temperatur der Reaktionszone(n) im Bereich von 20 bis 200°C und der Druck am Kopf der Kolonne im Bereich von 0,4 bis 5 bar liegen.

12. Verfahren nach einem der Ansprüche 1 bis 3, **dadurch gekennzeichnet, dass** die Alkylencarbonatverbindung(en) und der oder die Alkohol(e) im Molverhältnis von 1 : 2.0 bis 1 : 20 eingesetzt werden.

## Claims

1. Process for the continuous preparation of dialkyl carbonate of the formula (I)
(R¹O) ₂CO (I)
where R¹ is straight-chain or branched C₁-C₄-alkyl,
and of alkylene glycol of the formula (II)
R²(OH)₂ (II)
where R² is a C₂-C₄-alkyl,
as by-product by catalyzed transesterification of a cyclic alkylene carbonate with an alcohol of the formula (III)
R¹OH (III)
where R¹ is as defined above,
**characterized in that** the transesterification is carried out in countercurrent in a column with the cyclic alkylene carbonate (1) being introduced into the upper part of the column and an alcohol (3) containing dialkyl carbonate being introduced into the middle or lower part of the column and a further point of introduction for an alcohol-containing stream (4) being additionally provided below the point of introduction of the alcohol containing dialkyl carbonate,
where the ratio of the spacing between the point of introduction containing alkylene carbonate (1) and the point of introduction of the alcohol (3) containing dialkyl carbonate to the spacing between the point of introduction of alkylene carbonate (1) and the second point of introduction of alcohol (4) is from 0.20 to 0.52.

2. Process according to Claim 1, **characterized in that** the ratio of the spacing between the point of introduction of alkylene carbonate (1) and the point of introduction of the alcohol (3) containing dialkyl carbonate to the spacing between the point of introduction of alkylene carbonate (1) and the second point of introduction of alcohol (4) is from 0.28 to 0.44.

3. Process according to either Claim 1 or 2, **characterized in that** the dialkyl carbonate content of the alcohol (3) containing a dialkyl carbonate is preferably from 0.2 to 30% by weight.

4. Process according to any of Claims 1 to 3, **characterized in that** ethylene carbonate or propylene carbonate is used as cyclic alkylene carbonate.

5. Process according to any of Claims 1 to 4, **characterized in that** methanol is used as alcohol and dimethyl carbonate is used as dialkyl carbonate.

6. Process according to any of Claims 1 to 5, **characterized in that** alcohol having a purity of at least 90% by weight, preferably at least 95% by weight and particularly preferably at least 99.5% by weight, is introduced at the second, bottommost point of introduction of alcohol (4).

7. Process according to any of Claims 1 to 6, **characterized in that** the reaction is carried out in the presence of a homogeneous catalyst.

8. Process according to any of Claims 1 to 7, **characterized in that** potassium hydroxide or sodium hydroxide is used as catalyst.

9. Process according to any of Claims 1 to 8, **characterized in that** the transesterification column contains at least one enrichment section in the upper part of the column and at least one reaction zone below the enrichment section.

10. Process according to any of Claims 1 to 9, **characterized in that** the transesterification column has at least one stripping section below a reaction zone.

11. Process according to any of Claims 1 to 10, **characterized in that** the temperature of the reaction zone(s) is in the range from 20 to 200°C and the pressure at the top of the column is in the range from 0.4 to 5 bar.

12. Process according to any of Claims 1 to 3, **characterized in that** the alkylene carbonate compound(s) and the alcohol(s) are used in a molar ratio of from 1 : 2.0 to 1 : 20.

## Revendications

1. Procédé de fabrication continue de carbonate de dialkyle de formule (I)
(R¹O)₂CO (I)
dans laquelle R¹ représente un radical en C₁-C₄ linéaire ou ramifié,
et d'alkylène glycol de formule (II) en tant que produit secondaire
R²(OH)₂ (II)
dans laquelle R² représente un alkyle en C₂-C₄,
par transestérification d'un carbonate d'alkylène cyclique avec un alcool de formule (III)
R¹OH (III)
dans laquelle R¹ a la signification précédente, en présence d'un catalyseur,
**caractérisé en ce que** la transestérification est réalisée dans une colonne à contre-courant en introduisant le carbonate d'alkylène cyclique (1) dans la partie supérieure de la colonne et un alcool (3) contenant un carbonate de dialkyle dans la partie centrale ou inférieure de la colonne, et en prévoyant en plus un emplacement d'introduction supplémentaire pour un courant (4) contenant un alcool en dessous de l'introduction de l'alcool contenant un carbonate de dialkyle, le rapport entre la distance entre l'emplacement d'introduction du carbonate d'alkylène (1) et l'emplacement d'introduction de l'alcool (3) contenant un carbonate de dialkyle et la distance entre l'emplacement d'introduction du carbonate d'alkylène (1) et le second emplacement d'introduction d'un alcool (4) étant de 0,20 à 0,52.

2. Procédé selon la revendication 1, **caractérisé en ce que** le rapport entre la distance entre l'emplacement d'introduction du carbonate d'alkylène (1) et l'emplacement d'introduction de l'alcool (3) contenant un carbonate de dialkyle et la distance entre l'emplacement d'introduction du carbonate d'alkylène (1) et le second emplacement d'introduction d'un alcool (4) est de 0,28 à 0,44.

3. Procédé selon l'une quelconque des revendications 1 ou 2, **caractérisé en ce que** la teneur en carbonate de dialkyle dans l'alcool (3) contenant un carbonate de dialkyle est de préférence de 0,2 à 30 % en poids.

4. Procédé selon l'une quelconque des revendications 1 à 3, **caractérisé en ce que** du carbonate d'éthylène ou de propylène est utilisé en tant que carbonate d'alkylène cyclique.

5. Procédé selon l'une quelconque des revendications 1 à 4, **caractérisé en ce que** du méthanol est utilisé en tant qu'alcool et du carbonate de diméthyle en tant que carbonate de dialkyle.

6. Procédé selon l'une quelconque des revendications 1 à 5, **caractérisé en ce qu'**un alcool pur au moins à 90 % en poids, de préférence au moins à 95 % en poids et de manière particulièrement préférée au moins à 99,5 % en poids est introduit dans le second emplacement d'introduction d'alcool le plus inférieur (4).

7. Procédé selon l'une quelconque des revendications 1 à 6, **caractérisé en ce que** la réaction est réalisée en présence d'un catalyseur homogène.

8. Procédé selon l'une quelconque des revendications 1 à 7, **caractérisé en ce que** de l'hydroxyde de potassium ou de sodium est utilisé en tant que catalyseur.

9. Procédé selon l'une quelconque des revendications 1 à 8, **caractérisé en ce que** la colonne de transestérification contient au moins une zone d'enrichissement dans la partie supérieure de la colonne et au moins une zone de réaction sous la zone d'enrichissement.

10. Procédé selon l'une quelconque des revendications 1 à 9, **caractérisé en ce que** la colonne de transestérification comprend au moins une zone de rectification sous la zone de réaction.

11. Procédé selon l'une quelconque des revendications 1 à 10, **caractérisé en ce que** la température de la ou des zones de réaction se situe dans la plage allant de 20 à 200 °C et la pression à la tête de la colonne se situe dans la plage allant de 0,4 à 5 bar.

12. Procédé selon l'une quelconque des revendications 1 à 3, **caractérisé en ce que** le ou les composés de carbonate d'alkylène et le ou les alcools sont utilisés en un rapport molaire de 1:2,0 à 1:20.
